# EUROPEAN PATENT SPECIFICATION

(11) **EP 4 129 263 B1**
(45) Date of publication and mention of the grant of the patent: **28.05.2025**
(21) Application number: 21779562.4
(22) Date of filing: 31.03.2021
(51) Int. Cl.: A61H 3/00, A61F 5/01, A61B 5/11, A61B 5/103, A61H 1/02

(54) **ROBOTIC LEG ORTHOSIS FOR GAIT REHABILITATION TRAINING, METHOD FOR CONTROLLING SAME, AND AUTONOMOUS DRIVING LUGGAGE CARRIER**
ROBOTISCHE BEINORTHESE FÜR GANGREHABILITATIONSTRAINING, VERFAHREN ZUR STEUERUNG DAVON UND AUTONOM FAHRENDER GEPÄCKTRÄGER
ORTHÈSE DE JAMBE ROBOTISÉE POUR L'ENTRAÎNEMENT À LA RÉÉDUCATION DE LA MARCHE, SON PROCÉDÉ DE COMMANDE, ET PORTE-BAGAGES À ENTRAÎNEMENT AUTONOME

(30) Priority: 31.03.2020 KR 20200039397
(43) Date of publication of application: 08.02.2023
(73) Proprietor: Seoul National University Hospital, Seoul 03080 (KR); Seoul National University R & DB Foundation, Seoul 08826 (KR)
(72) Inventor: PARK, Jihong, Seongnam-si, Gyeonggi-do 13434 (KR); LIM, Jae-Young, Seoul 07340 (KR); PARK, Yong-Lae, Seoul 06289 (KR); KWON, Junghan, Seoul 08816 (KR)
(74) Representative: V.O.
(86) International application number: PCT/KR2021/004013
(87) International publication number: WO 2021/201605

(56) References cited:
- EP-B1- 2 825 146
- ES-T3- 2 253 558
- KR-A- 20130 056 026
- KR-A- 20160 057 034
- KR-B1- 100 984 063
- KR-B1- 100 984 063
- KR-Y1- 200 352 095
- US-A1- 2012 089 063
- US-A1- 2012 089 063
- US-A1- 2019 336 315

## Description

### Technical Field

The present specification relates to a robotic leg orthosis for gait rehabilitation training, a robotic orthosis for rehabilitation training and a method for controlling same.

### Background Art

Stroke is one of the main causes of death not only in Korea but also throughout the world, one of the main causes of adult disability, and a disease that causes a decline in gait, daily living activities, and cognition and lowers the quality of life, resulting in a large burden personally and socially to a patient A reduction in stroke mortality due to the medical advancement results in an increase in the number of patients suffering from sequelae of a cranial nerve disease.

For a lower extremity, hemiplegia causes a lack of physical (muscular) strength of an affected leg for supporting a patient's weight during walking, thus reducing gait stability, a lack of strength for generating thrust by kicking the ground, and a phenomenon of foot drop during a swing motion.

As a result, the patient suffers from gait abnormality which is characterized by hip hiking and circumduction gait as recompense motions. Asymmetry between both lower extremities not only disturbs gait rehabilitation training for a normal pattern but also is a principal cause of exhaustion of physical strength during walking and an increase in fall risk of a patient.

Currently, various types of orthoses are used in rehabilitation courses for hemiplegia patients. A fixed ankle orthosis for gait assistance can inhibit an ankle from excessive dropping, but does not sufficiently imitate motions of a joint during normal gait motions of a lower extremity, thus being a cause of degrading gait stability and symmetry.

In order to supplement such shortcomings described above, several items of equipment have been developed by adopting robot engineering for over a decade, and rehabilitation or the like using a robot having an exoskeleton structure is reported to have a partial clinical effect; however, most of the items of equipment are high in cost and large in volume, are often used only for repeating a simple motion, and have a problem of a limit in detecting a motion of a patient and providing a customized treatment protocol.

In addition, an existing orthosis or a rehabilitation apparatus does not include a sensing system suitable for accurately detecting a patient's gait condition, and thus an additional measurement instrument needs to be used in order to quantitatively analyze characteristics of the patient's gait and motion.

Consequently, it is not possible to provide feedback based on accurate evaluation of a condition, and thus it is difficult to perform quantitative rehabilitation training.

Such problems described above bring about a demand for a device that can assist a post-stroke patient's knee joint and ankle joint strength on one leg.

### [Prior Art Literature]

### [Patent Literature]

[Patent Literature 1] Korean Patent Registration No. 10-2018175 US 2019/336315 A1 describes a robotic leg orthosis for gait rehabilitation training, according to the preamble of claim 1.

EP 2 825 146 B1 describes a robotic orthosis for rehabilitation training according to the preamble of claim 13.

### Disclosure

### Technical Problem

According to an aspect of embodiments of the present invention, there are provided a robotic leg orthosis for gait rehabilitation training and a method for controlling same, in which the robotic leg orthosis can assist a rehabilitation patient's knee joint on one leg and/or ankle joint strength according to a gait cycle of the rehabilitation patient, and can be implemented as a soft wearable device.

According to another aspect of the embodiments of the present invention, there are provided a mobile robotic orthosis for gait rehabilitation training that is not stationary but can be applied during a movement process of the patient, and a luggage carrier applicable thereto.

### Solution to Problem

According to exemplary embodiments of the present invention, there is provided a robotic leg orthosis for gait rehabilitation training including: an orthosis that is installed on at least one of a knee, an ankle joint, a shin, and a calf of an affected leg which is a rehabilitation target. The orthosis comprises: a sleeve surrounding at least one of the knee, the ankle joint, the shin, and the calf; and an air chamber which is mounted so as to be connected to the sleeve and provides assisting strength to at least one of the knee, the ankle joint, the shin, and the calf. The air chamber may provide assisting strength corresponding to a patient's gait cycle.

According to an embodiment, the orthosis may include a first ankle supporting member that is installed at an ankle so as to inhibit inversion or eversion bending of the ankle, and the first ankle supporting member may have: an ankle sleeve surrounding an ankle joint; and ankle supporting chambers which are air chambers mounted to be connected to the ankle sleeve, and which, when air is introduced therein to inflate the air chambers, support the ankle joint so as to inhibit inversion or eversion bending of the ankle.

According to the embodiment, the air chambers may surround both inversion and eversion sides of an ankle, respectively. Air may be injected into the air chambers before the affected leg reaches ground, and the air chambers may be inflated to provide assisting strength so as to inhibit inversion or eversion bending of the ankle. The air chambers may maintain pressure therein in a stance phase to provide the assisting strength so as to inhibit inversion or eversion bending of the ankle and maintain a stretched state.

According to the embodiment, the orthosis may include a second ankle supporting member that is installed on at least one of a shin and a calf such that an ankle is flexed toward a dorsum or a sole of a foot. The second ankle supporting member may further have: a shin sleeve surrounding the shin; guards that are installed at both the shin and the calf, respectively; and artificial muscle packs that are connected to the respective guards and have one or more air chambers inside.

According to the embodiment, the second ankle supporting member may further have: a Velcro tie that surrounds and secures the guards; and a buckle that is installed at a foot accommodating portion and combines the artificial muscle packs.

According to the embodiment, the air chambers may be installed at both sides of the shin and the calf, respectively. In a state in which a sole is in contact with ground, air in the air chamber installed at the side of the shin may be discharged outside, and air may be injected into the air chamber installed at the side of the calf. In a state in which a sole is separated from the ground, air may be injected into the air chamber installed at the side of the shin, and air in the air chamber installed at the side of the calf may be discharged outside.

According to the embodiment, the orthosis may further include a knee stretching member which is provided to be installable on a knee so as to provide assisting strength for enabling a knee to be stretched. The knee stretching member may have: a knee sleeve surrounding a knee j oint; and knee supporting chambers which are air chambers mounted to be connected to the knee sleeve, and which, when air is introduced therein to inflate the air chambers, enable the knee to be stretched by supporting the knee joint.

According to the embodiment, the air chambers may be located at both sides of a rear surface portion of the knee joint, respectively.

According to the embodiment, the knee stretching member may enable the knee joint to be stretched in a swing phase and may enable a state in which the knee is stretched to be maintained in a stance phase.

According to the embodiment, the sleeve may be made of an elastic material, and the air chambers may be made of a cloth material coated with a soft material such as elastomer such as polyurethane.

In addition, according to the exemplary embodiments of the present invention, there is provided a method for controlling the robotic leg orthosis for gait rehabilitation training described above, the method including: a step of checking at least one of a rehabilitation patient's gait condition and gait pattern in real time based on an angle between ground and at least one of the patient's thigh, shin, and dorsum of a foot during the rehabilitation patient's walking, by an inertial measurement module; a step of determining the patient's gait cycle as any one of a loading response phase, a mid stance phase, a terminal stance phase, a pre-swing phase, an initial swing phase, a mid swing phase, and a terminal swing phase, based on at least one of the gait condition and the gait pattern, by a main controller; a step of causing a knee stretching member to enable a knee joint to be stretched in the terminal swing phase and enable a state in which the knee is stretched to be maintained for the loading response phase to the terminal stance phase according to the determined gait cycle, by the main controller; a step of causing a first ankle supporting member to start supporting an ankle so as to inhibit inversion or eversion bending of an ankle joint in the terminal swing phase and maintain ankle support so as to inhibit the inversion or eversion bending of the ankle joint for the loading response phase to the terminal stance phase according to the determined gait cycle, by the main controller; and a step of causing a second ankle supporting member to assist dorsiflexion for the initial swing phase to the terminal swing phase, assist dorsiflexion in the loading response phase, and assist plantarflexion for the mid stance phase to the pre-swing phase according to the determined gait cycle, by the main controller. The inertial measurement module may be installed on at least one of the patient's thigh, shin, and dorsum of a foot, and the main controller may be contained in the luggage carrier.

In addition, according to the exemplary embodiments of the present invention, there is provided a robotic orthosis for rehabilitation training including: an air chamber that assists a rehabilitation body; and a luggage carrier that generates compressed air and supplies the generated compressed air to the air chamber. The luggage carrier is an autonomous driving luggage carrier.

According to another embodiment, the luggage carrier may have: an air compressor which generates compressed air; an air tank which stores the generated compressed air; and a pressure regulator which regulates pressure of the compressed air.

According to the other embodiment, the luggage carrier may have a laser distance sensor which is capable of sensing a distance between a patient and a camera that is capable of imaging a location of the patient such that the luggage carrier is capable of performing autonomous driving so as to follow the patient by recognizing the location of the patient.

According to the other embodiment, the robotic orthosis for rehabilitation training may further include an inertial measurement module that is installed on at least one of the patient's thigh, shin, and dorsum of a foot. The luggage carrier may further include a main controller. The steps of the method for controlling the robotic leg orthosis for gait rehabilitation training described above may be performed by the inertial measurement module and the main controller.

### Advantageous Effects of Invention

A robotic leg orthosis for gait rehabilitation training according to the embodiments of the present invention is made of a soft material so as to be lightweight, flexible, well wearable, and safe.

In addition, the robotic leg orthosis for gait rehabilitation training according to the embodiments of the present invention includes pneumatic air chambers or artificial muscle packs so as to be capable of providing active assisting strength when compressed air is injected, so it is possible to assist and support behavior of a knee and/or an ankle joint, when compressed air is injected.

In addition, the robotic leg orthosis for gait rehabilitation training according to the embodiments of the present invention can provide a luggage carrier which can perform autonomous driving and supply air pressure, thereby performing rehabilitation training not in a stationary manner but in a movable manner.

In addition, the robotic leg orthosis for gait rehabilitation training according to the embodiments of the present invention can obtain the patient's gait condition and gait pattern in real time and can perform real-time control to operate the orthosis according to a special gait point and phase.

Further, the robotic leg orthosis for gait rehabilitation training according to the embodiments of the present invention can be applied to customized rehabilitation through feedback of quantitative gait data and gait indexes by acquiring and analyzing a patient's gait data.

According to the embodiments of the present invention described above, the robotic leg orthosis can assist a patient in a natural gait close to a normal gait, can assist a patient in daily life, and can maximize rehabilitation effects.

### Brief Description of Drawings

FIG. 1 is a perspective view illustrating an example of a robotic leg orthosis for gait rehabilitation training put on a user according to an embodiment of the present invention.
FIG. 2 is a plan view illustrating a structure of a knee stretching member according to the embodiment of the present invention.
FIG. 3A is a conceptual view illustrating an operation of the knee stretching member in a initial and mid swing phase of the knee stretching member according to the embodiment of the present invention.
FIG. 3B is a conceptual view illustrating an operation of the knee stretching member in a terminal swing phase of the knee stretching member according to the embodiment of the present invention.
FIG. 3C is a conceptual view illustrating an operation of the knee stretching member in a stance phase of the knee stretching member according to the embodiment of the present invention.
FIG. 4 is a plan view illustrating a first ankle supporting member according to the embodiment of the present invention.
FIG. 5A is a conceptual view illustrating a state in which air is yet to be injected into the first ankle supporting member according to the embodiment of the present invention.
FIG. 5B is a conceptual view illustrating a state in which air is injected into the first ankle supporting member according to the embodiment of the present invention.
FIG. 5C is a conceptual view illustrating an example in which the first ankle supporting member supports an ankle at an inversion side according to the embodiment of the present invention.
FIG. 5D is a conceptual view illustrating an example in which the first ankle supporting member supports the ankle at an eversion side according to the embodiment of the present invention.
FIG. 6 is a plan view illustrating an artificial muscle pack according to the embodiment of the present invention.
FIG. 7A is a conceptual view illustrating an example of a state in which air is yet to be injected into air chambers at both sides of a shin and a calf according to the embodiment of the present invention.
FIG. 7B is a conceptual view illustrating an example of a state in which air is injected into the air chamber at the side of the shin according to the embodiment of the present invention.
FIG. 7C is a conceptual view illustrating an example of a state in which air is injected into the air chamber at the side of the calf according to the embodiment of the present invention.
FIG. 8 is a perspective view illustrating a luggage carrier according to the embodiment of the present invention.
FIG. 9 is a block diagram illustrating a configuration in the luggage carrier according to the embodiment of the present invention.
FIG. 10 is a conceptual view illustrating an example in which an inertial measurement module and an insole module are installed according to the embodiment of the present invention.
FIG. 11 is a conceptual diagram illustrating angles between ground and a thigh, a shin, and a dorsum of a foot, angles of a hip, a knee, and an ankle joint, and locations of a knee, an ankle, and a tiptoe according to the embodiment of the present invention.
FIG. 12 is a table illustrating generation of an algorithm and a control force in each gait phase according to the embodiment of the present invention.
FIG. 13 illustrates photographs displaying a wearing order of the robotic leg orthosis for gait rehabilitation training according to an embodiment of the present invention.

### [Description of Reference Numbers]

100: Robotic leg orthosis for gait rehabilitation training
10: Knee stretching member
13: Knee sleeve
15: Knee supporting chamber
18: Velcro
20: First ankle supporting member
21: Ankle sleeve
25: Ankle supporting chamber
28: Velcro
30: Second ankle supporting member
31: Shin sleeve
32: Guard
34: Artificial muscle pack
40: Luggage carrier
41: Handle
41a: Camera
41b: Laser distance sensor
42: Air compressor
43: Air tank
44: Pressure regulator
45: Battery
46: Loadcell amplifier
47: IMU receiver
48: Main controller
49: Self-driving unit
50: Inertial measurement module
60: Insole module
70: Air hose
71: Waist belt

### Mode for Invention

Hereinafter, embodiments disclosed in this specification will be described in detail with reference to the accompanying drawings; however, the same or similar reference signs are assigned to the same or similar configurational elements, and the repeated description thereof is to be omitted.

In the description of the embodiments disclosed in this specification, when the specific description of a known technology related to the embodiments is considered to obscure the gist of the embodiments disclosed in this specification, the detailed description thereof is to be omitted. In addition, the accompanying drawings are only provided to enable the embodiments disclosed in this specification to be easily understood, and thus the technical idea disclosed in this specification is not limited to the accompanying drawings. All modifications, equivalents, and alternatives included in the technical idea and technical scope of the present invention are to be construed to be part of the present invention.

Terms having an ordinal number such as first or second can be used in describing various configurational elements; however, the configurational elements are not limited to the terms. The terms are used only for a purpose of distinguishing one configurational element from another configurational element.

The description in which one configurational element is mentioned to be "connected to" another configurational element is to be understood to mean that the one configurational element can be directly connected to the other configurational element or that still another configurational element can be present therebetween.

Singular expression also includes plural expression thereof, unless obviously implied otherwise in context.

In this specification, a term such as "to comprise" or "to have" is construed to specify that a feature, a number, a step, an operation, a configurational element, a part, or an assembly thereof described in the specification is present and not to exclude a possibility of presence or addition of one or more other features, numbers, steps, operations, configurational elements, parts, or assemblies thereof in advance.

In this specification, a term such as "unit", "module", or "controller" which includes a control process can indicate not only hardware but also an assembly of hardware and software driven by the hardware. For example, the hardware can be a data processing unit including a CPU or another processor. In addition, the software driven by the hardware can be a program such as a running process, an object, an executable file, a thread of execution, or a computational program.

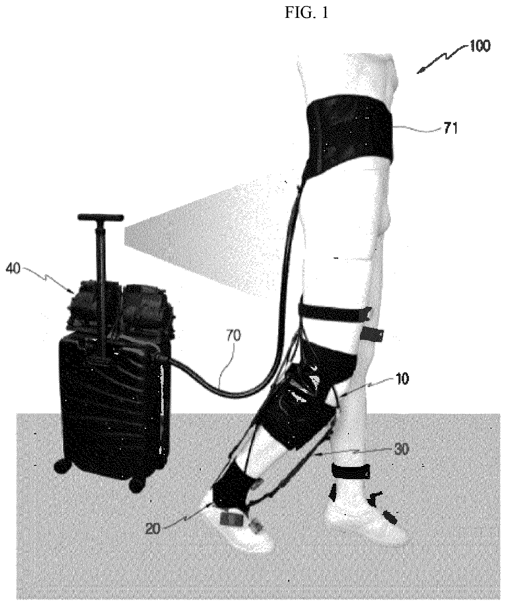

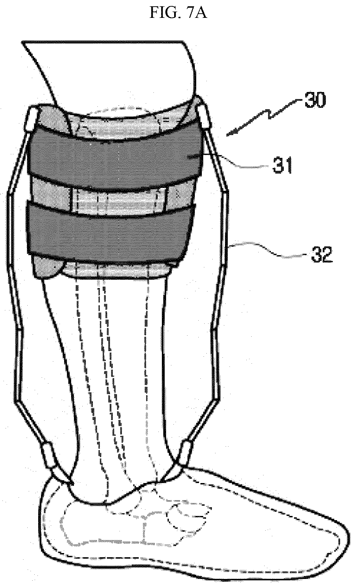

With reference to FIGS. 1 to 7C, a robotic leg orthosis 100 for a lower extremity for gait rehabilitation training according to the embodiments of the present invention is described.

The robotic leg orthosis 100 for gait rehabilitation training according to the embodiments includes an orthosis that is installed on at least one of a knee, an ankle joint, a shin, and a calf of an affected leg which is a rehabilitation target.

The orthosis includes a sleeve surrounding at least one of the knee, the ankle joint, the shin, and the calf, and an air chamber which is mounted so as to be connected to the sleeve and provides assisting strength to at least one of the knee, the ankle joint, the shin, and the calf. The air chamber can provide assisting strength corresponding to a patient's gait cycle.

The orthosis can be an ankle supporting member or a knee stretching member, for example. The specific description thereof will be provided.

In an embodiment, the robotic leg orthosis 100 for gait rehabilitation training of the present invention can include a first ankle supporting member 20.

The first ankle supporting member 20 is installed at an ankle so as to inhibit inversion or eversion bending of the ankle.

The first ankle supporting member 20 includes an ankle sleeve 21 and an ankle supporting chamber 25.

The ankle sleeve 21 is provided to surround an ankle joint. Desirably, the ankle sleeve 21 is made of an elastic material.

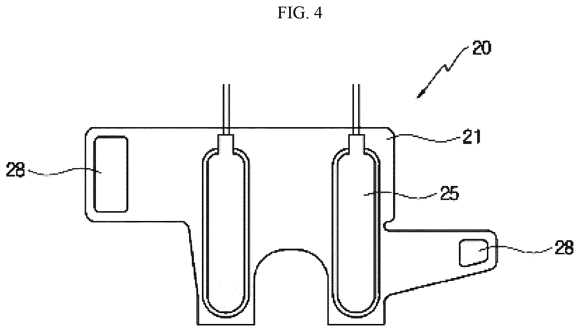

The ankle sleeve 21 can have patches of Velcro 28 at both ends thereof, and the patches of Velcro 28 enable the ankle sleeve 21 to surround and fix the patient's ankle joint. FIG. 4 illustrates an example in which one patch of Velcro 28 is attached to each of right and left ends of the ankle sleeve 21.

Positions of the patches of Velcro 28 on the ankle sleeve 21 or positions at which the patches of Velcro 28 are attached to each other can be adjusted depending on a body size.

The ankle supporting chamber 25 can be mounted to be connected to the ankle sleeve 21 and can support the ankle joint when air is injected therein and is inflated.

The inflated ankle supporting chamber 25 has an increase in rigidity so as to inhibit excessive inversion or eversion bending of the ankle.

Before the affected leg, on which the first ankle supporting member 20 is installed, reaches the ground, air is injected into the ankle supporting chamber 25 and to inflate the ankle supporting chamber in advance, and thereby rigidity of the ankle in inversion and eversion directions can be reinforced. In this manner, the impact applied to the ankle in the initial stance phase in which the affected leg comes into contact with the ground is absorbed, and gait stability is improved.

In addition, the first ankle supporting member 20 enables the pressure of the ankle supporting chamber 25 to be maintained in the stance phase so as to maintain a state in which the ankle is straightly stretched such that the affected leg can support the weight and a sprain and a falling accident due to bending of the ankle can be prevented.

Intensity of an ankle supporting force of the first ankle supporting member 20 which supports the ankle can be controlled by adjusting flexural rigidity of ankle support based on pressure intensity of air injected into the ankle supporting chamber 25.

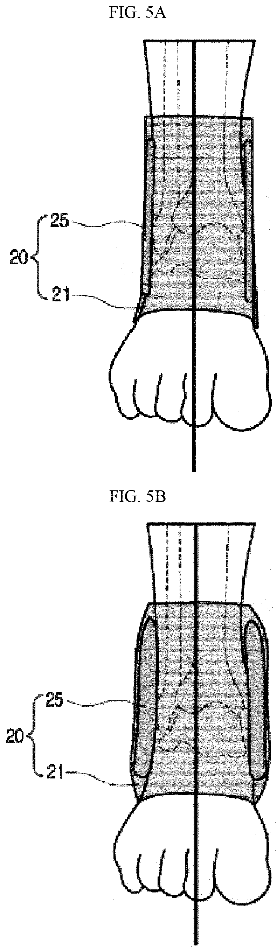

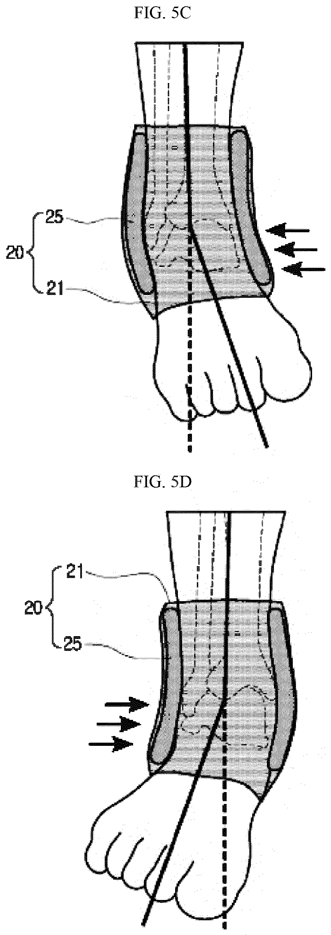

FIGS. 5C and 5D illustrate an example in which the first ankle supporting member 20 supports the ankle at inversion and eversion sides.

The ankle supporting chamber 25 can be manufactured of a cloth material and, desirably, can be manufactured of a cloth material coated with polyurethane.

In addition, the robotic leg orthosis 100 for gait rehabilitation training of the embodiments of the present invention can further include a second ankle supporting member 30.

The second ankle supporting member 30 can include a shin sleeve 31, guards 32, and artificial muscle packs 34.

The shin sleeve 31 can be provided to surround a shin.

The shin sleeve 31 can have patches of Velcro at both ends thereof, and the patches of Velcro enable shin sleeve 31 to surround and fix the vicinity of the patient's shin.

The guards 32 can be installed at both the shin and a calf, respectively.

The guards 32 can be attached to fix an upper part of the artificial muscle packs 34 on the shin sleeve 31. Desirably, the guard 32 is made of a hard material. The guard 32 can have a Velcro tie such that the Velcro tie can be tied on the shin and the calf, and thereby the guard 32 can be fixed.

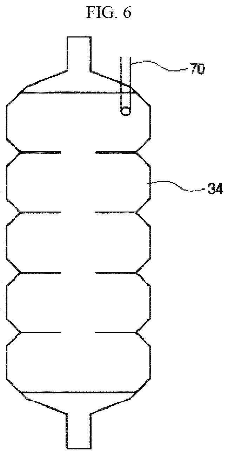

The artificial muscle packs 34 can be connected to the guards 32 and can have a plurality of air chambers therein as illustrated in FIG. 6.

The air chambers can be installed at both sides of the shin and the calf.

The air chambers can be provided in plurality and can be connected in series in a direction parallel to the leg. The air chamber maintains a flat shape when air is discharged. When compressed air is injected into the air chamber, the air chamber is inflated and is contracted in a length direction thereof so as to generate a protective force at the calf and the shin.

The artificial muscle pack 34 can be manufactured of a cloth material coated with polyurethane.

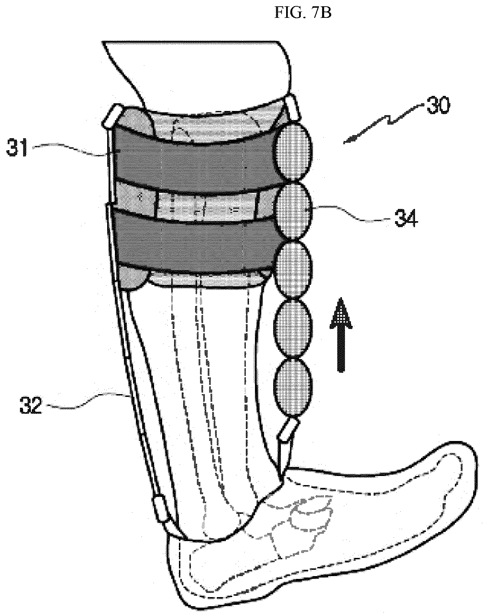

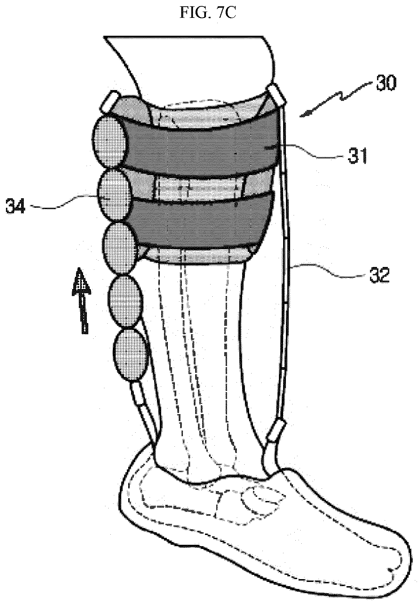

The artificial muscle packs 34 can have the air chambers at the sides of the shin and the calf. The artificial muscle packs 34, with reference to FIGS. 7A and 7B, cause the air chamber installed at the side of the shin to assist a motion of dorsiflexion of a foot and, with reference to FIG. 7C, cause the air chamber installed at the side of the calf to assist plantarflexion.

The artificial muscle packs 34 assist the motion of the dorsiflexion of the ankle joint so as to inhibit foot drop and enable sufficient foot clearance to be secured by injecting the compressed air into the air chamber installed at the side of the shin in the swing phase and inflating the air chambers.

In addition, the artificial muscle packs 34 assist the motion of the dorsiflexion even during initial contact, which is a time when the foot starts to reach the ground, so as to absorb and soften the impact occurring when the foot reaches the ground.

On the other hand, air in the air chamber attached to the shin is emitted in the stance phase, and the compressed air is injected into the air chamber attached to the calf on a rear surface of the leg such that the air chamber is inflated to assist the motion of the plantarflexion of the ankle joint. Hence, while the foot kicks the ground, thrust is generated.

Intensity of a support force of the second ankle supporting member 30 which supports the shin and the calf can be controlled by adjusting flexural rigidity of shin and calf support based on pressure intensity of air injected into air chambers.

As an example, in a state in which a sole is in contact with the ground, air in the air chamber installed at the side of the shin can be discharged outside, and air can be injected into the air chamber installed at the side of the calf.

In addition, in a state in which a sole is separated from the ground, air can be injected into the air chamber installed at the side of the shin, and air in the air chamber installed at the side of the calf can be discharged outside.

The second ankle supporting member 30 can further include a Velcro tie and a buckle.

The Velcro tie can surround and fix the guards 32.

The buckle is installed at a foot accommodating portion and combines the artificial muscle packs 34.

In addition, the robotic leg orthosis 100 for gait rehabilitation training of the embodiments of the present invention can further include a knee stretching member 10.

The knee stretching member 10 can be provided to be installable on a knee.

In addition, the knee stretching member 10 enables a knee joint to be stretched in the swing phase and enables a state in which the knee is stretched to be maintained in a stance phase, thus, enabling an affected leg to support body weight.

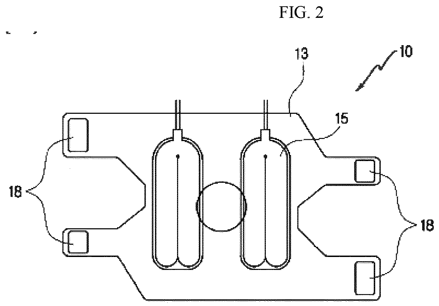

As illustrated in FIG. 2, the knee stretching member 10 can include a knee sleeve 13 and a knee supporting chamber 15.

The knee sleeve 13 can be provided to surround the knee joint. Desirably, the knee sleeve 13 is made of an elastic material.

The knee sleeve 13 can have patches of Velcro 18 at both ends thereof, and the patches of Velcro 18 enable the knee sleeve 13 to surround and fix a leg around a patient's knee joint. FIG. 2 illustrates an example in which the two patches of Velcro 18 are attached to each of right and left ends of the knee sleeve 13.

Positions of the patches of Velcro 18 or positions at which the patches of Velcro 18 are attached to each other can be adjusted depending on a body size.

The knee supporting chamber 15 is mounted so as to be connected to the knee sleeve 13, and when air is introduced therein to inflate the knee supporting chamber in the swing phase, enables the knee to be stretched by supporting the knee joint, and enables the state in which the knee is stretched to be maintained in the stance phase.

The knee supporting chamber 15 can be manufactured of a cloth material and, desirably, can be manufactured of a cloth material coated with polyurethane.

When air is injected into the knee supporting chamber 15, the knee supporting chamber 15 is inflated so as to support the knee joint. In addition, when the air injected into the knee supporting chamber 15 is discharged, the knee supporting chamber is deformed into a flat shape and does not affect movement of the knee joint.

In a state in which air is injected into the knee supporting chamber 15 to inflate the knee supporting chamber, and the knee supporting chamber 15 is inflated and is tight while being deformed into a bar shape.

When the knee supporting chamber 15 is inflated in the terminal swing phase, the knee supporting chamber 15 enables the knee joint to be extended in a direction parallel to the leg.

In addition, in the stance phase, the knee supporting chamber 15 maintains pressure therein to enable the state in which the leg is straightly stretched to be maintained such that the affected leg can support the weight. In addition, the knee supporting chamber 15 assists rigidity of the knee j oint, thereby enabling improvement of gait stability and injury prevention in the stance phase.

A degree of assisting strength of the knee stretching member 10 can be controlled by adjusting flexural rigidity of the knee supporting chamber 15 based on the pressure intensity of air injected into the knee supporting chamber 15.

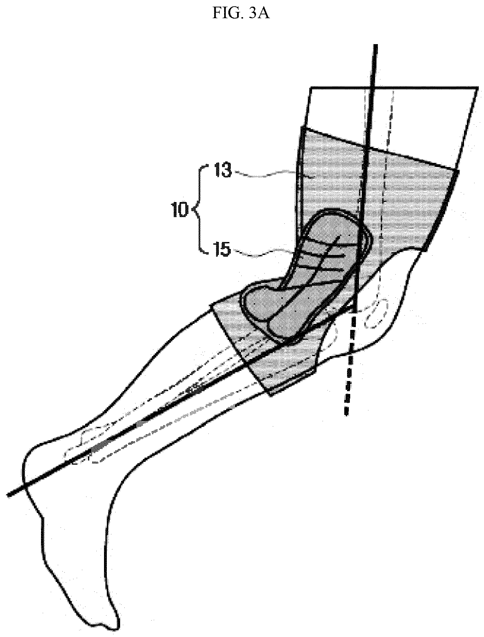

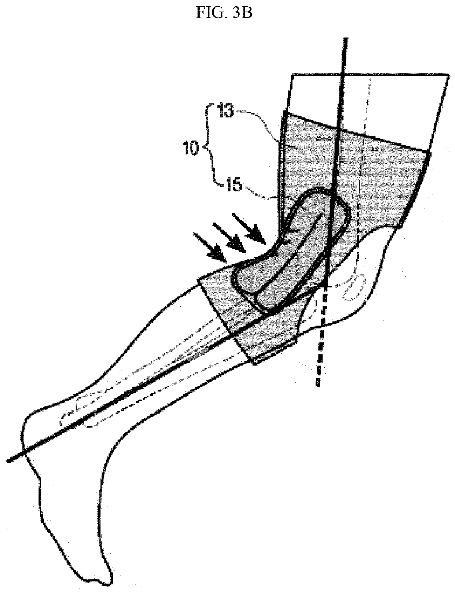

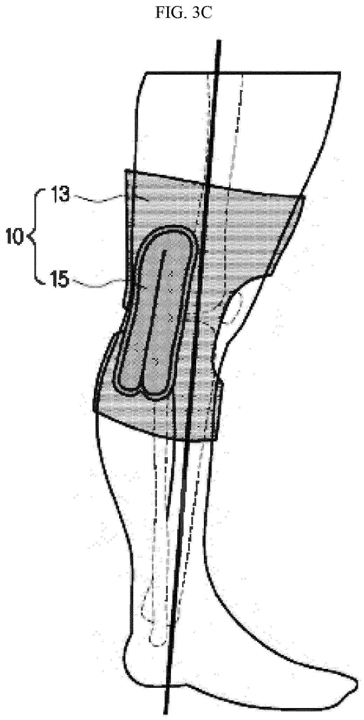

FIGS. 3A to 3C illustrate an example in which air is not injected into the knee supporting chamber 15 in the initial and mid swing phase and the knee supporting chamber 15 is inflated in the direction parallel to the leg to assist stance in the terminal swing phase and enables the state in which the leg is straightly stretched to be maintained in the stance phase.

On the other hand, according to the embodiments of the present invention, there is provided a robotic leg orthosis for rehabilitation training, the robotic orthosis including: an air chamber that assists a rehabilitation body; and a luggage carrier that generates compressed air and supplies the generated compressed air to the air chamber. The luggage carrier is an autonomous driving luggage carrier.

In the embodiment, the robotic leg orthosis 100 for gait rehabilitation training described above can further include a luggage carrier 40.

The luggage carrier 40 can generate compressed air and can supply the generated compressed air to at least one of the knee stretching member 10 and the first and second ankle supporting members 30.

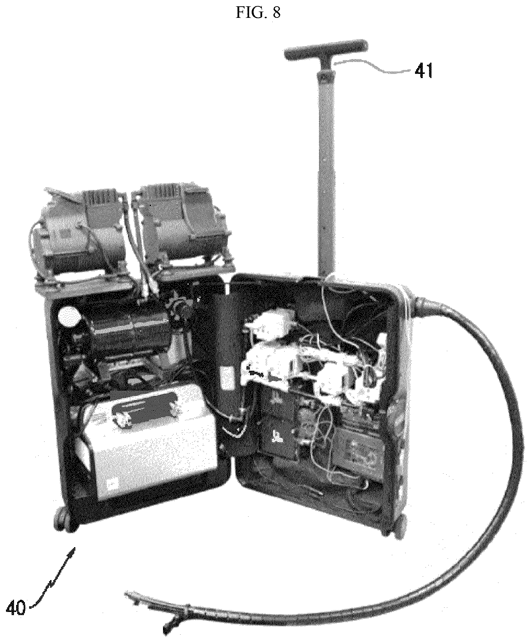

With reference to FIG. 8, the luggage carrier 40 can have a handle 41, and a camera 41a and a laser distance sensor 41b are installed on the handle 41 so as to enable the luggage carrier 40 to recognize a location of a patient and move by a self-driving unit 49 to follow the patient. Hence, the luggage carrier 40 according to the embodiment of the present invention can perform autonomous driving.

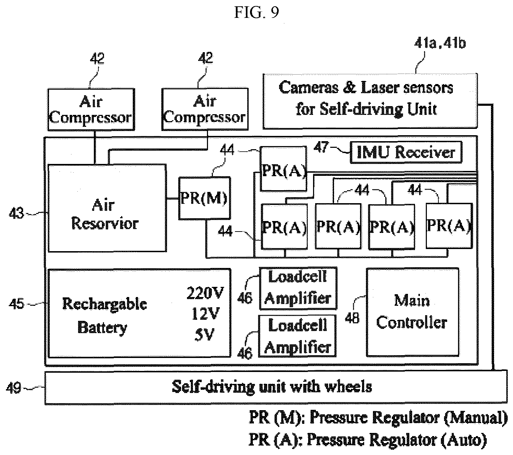

In addition, FIG. 9 is a block diagram illustrating a configuration in the luggage carrier 40. With reference to FIG. 9, the luggage carrier 40 contains an air compressor 42 that generates compressed air, an air tank 43 that stores the compressed air, a pressure regulator 44 that regulates intensity of air pressure, a battery 45 for electric power supply, a loadcell amplifier 46 for collecting a sensor signal, an IMU receiver 47, and a main controller 48.

The main controller 48 analyzes in real time the gait cycle and pattern from a signal measured in an inertial measurement module 50 to be described below and controls the pressure regulator 44 to supply the compressed air having set pressure to a driver at an appropriate time.

In addition, the main controller 48 monitors a gait condition by communicating with an external desktop or laptop and enables external control to be performed.

In addition, the robotic leg orthosis 100 for gait rehabilitation training according to the embodiment of the present invention can further include the inertial measurement module 50.

The inertial measurement module 50 checks the patient's gait condition and gait pattern in real time based on an angle between the ground and at least one of the thigh, the shin, and the dorsum of the foot during the patient's walking so as to enable the main controller 48 to control an operation of at least one of the knee stretching member 10 and the first and second ankle supporting members 30.

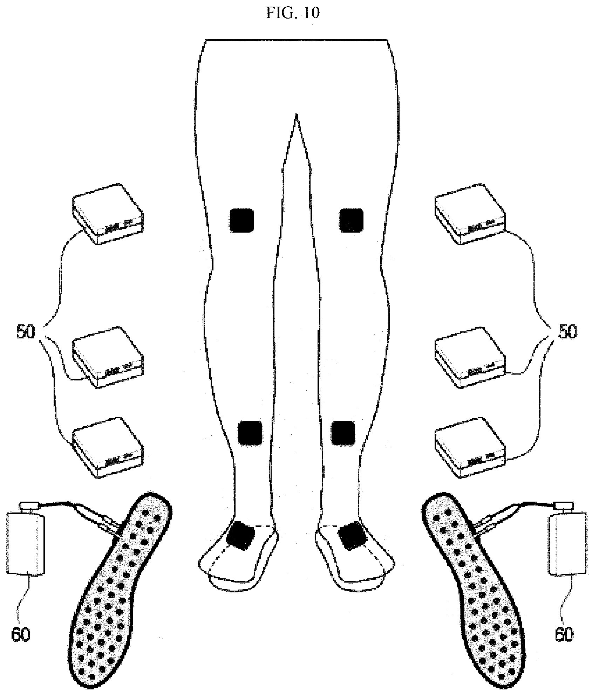

In this respect, with reference to FIG. 10, the inertial measurement module 50 can be installed on at least one of the patient's thigh, shin, and dorsum of the foot.

For example, the inertial measurement module 50 can be an inertial measurement unit (IMU).

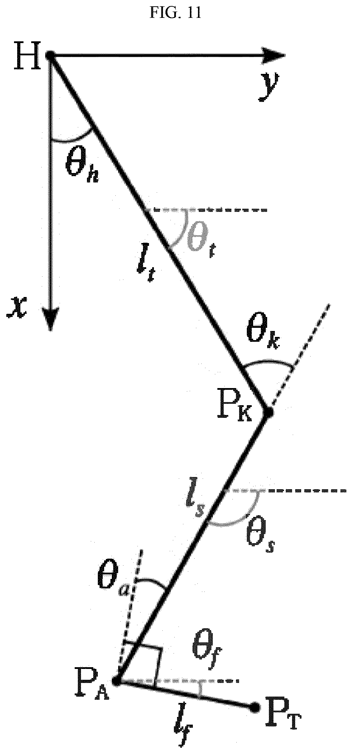

For example, with reference to FIG. 11, the inertial measurement module 50 can measure an angle θₜ, θₛ, or θ_{f} between the ground and at least one of the thigh, the shin, and the dorsum of the foot, and then can calculate an angle θₕ, θₖ, or θₐ of a hip, a knee, or an ankle joint by using a kinematics model of a lower extremity. In addition, with a location H of a hip joint as an origin point, locations P_{K}, P_{A}, and P_{T} of the knee, the ankle, and a tiptoe can be calculated.

On the other hand, the luggage carrier 40 can include a wireless plantar pressure insole module 60 so as to measure whether both soles are in contact with the ground in real time.

In addition, the inertial measurement module 50 enables the main controller 48 to determine a gait cycle as any one of a loading response phase, a mid stance phase, a terminal stance phase, a pre-swing phase, an initial swing phase, a mid swing phase, and a terminal swing phase based on a posture of the patient's both lower extremities and whether the patient's soles are in contact with the ground.

As described above, the luggage carrier 40 can supply the compressed air to the knee stretching member 10 in the terminal swing phase, the loading response phase, the mid stance phase, and the terminal stance phase and supply the compressed air to the first ankle supporting member 20 in the terminal swing phase, the loading response phase, and the terminal stance phase.

As described above, the patient's gait condition and gait pattern can be checked in real time by using sensing information measured and calculated in real time, and the patient's gait condition and gait pattern can be used in real-time control to operate pneumatic artificial muscle at a specific gait point and phase. Further, the patient's gait data can be acquired and analyzed so as to feedback quantitative gait data and gait indexes and thereby can be used in customized rehabilitation.

In addition, the robotic leg orthosis 100 for a lower extremity for gait rehabilitation training can further include an air hose 70 that enables the compressed air generated in the luggage carrier 40 to be transmitted to the knee stretching member 10, the first ankle supporting member 20, and second ankle supporting member 30.

One end side of the air hose 70 communicates with the air tank 43, and the other end side thereof communicates with each of the knee supporting chamber 15, the ankle supporting chamber 25, and the artificial muscle packs 34 so as to supply compressed air in the air tank 43 to each of the knee stretching member 10, the first ankle supporting member 20, and the second ankle supporting member 30.

The air hose 70 can include a waist belt 71 for branching into the knee supporting chamber 15, the ankle supporting chamber 25, and the artificial muscle packs 34, and the air hose 70 can be fixed to the patient's waist with the waist belt 71.

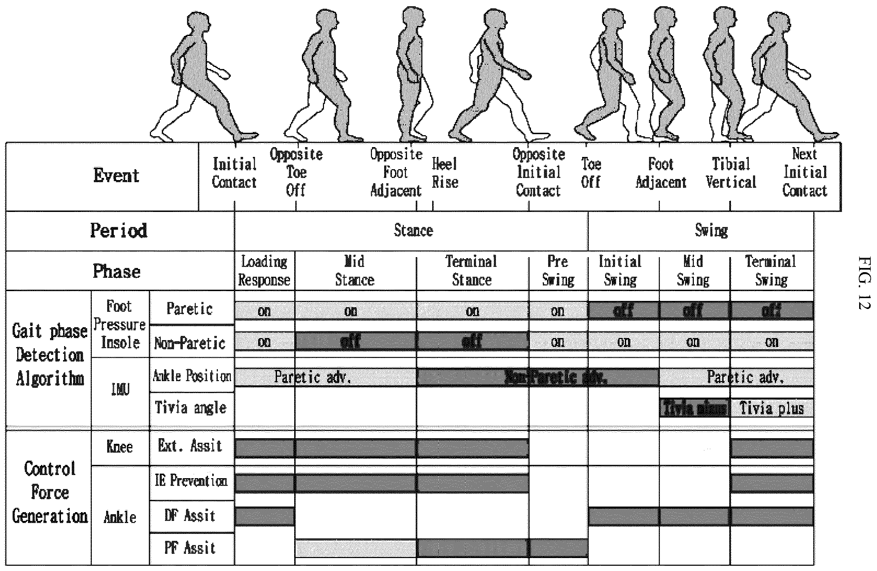

In the embodiment of the present invention, the gait phase will be described hereinafter. Regarding the gait phase, FIG. 12 illustrates generation of an algorithm and a control force in each gait phase.

The gait phase largely includes the stance phase in which a foot is in contact with the ground and the swing phase in which the foot is separated from the ground, and the gait phase includes seven phases in total depending on whether relative locations of both legs are in contact with the ground.

The seven phases of the stance phase and the swing phase are divided into a loading response phase, a mid stance phase, a terminal stance phase, a pre-swing phase, an initial swing phase, a mid swing phase, and a terminal swing phase.

A phase to which a gait condition belongs during walking can be determined in real time by detecting posture of both lower extremities and whether the soles are in contact with the ground by using the inertial measurement module 50 included in the embodiment of the present invention, and determination criteria are as follows.

The loading response phase corresponds to a case where both feet are in contact with the ground, and an affected ankle joint is located in front of an unaffected ankle joint.

The mid stance phase corresponds to a case where an affected foot is in contact with the ground, an unaffected foot is separated from the ground, and the affected ankle joint is located in front of the unaffected ankle joint.

The terminal stance phase corresponds to a case where the affected foot is in contact with the ground, the unaffected foot is separated from the ground, and the affected ankle joint is located behind the unaffected ankle joint.

The pre-swing phase corresponds to a case where both feet are in contact with the ground, and the affected ankle joint is located behind the unaffected ankle joint.

The initial swing phase corresponds to a case where the affected foot is separated from the ground, the unaffected foot is in contact with the ground, and the affected ankle joint is located behind the unaffected ankle joint.

The mid swing phase corresponds to a case where the affected foot is separated from the ground, the unaffected foot is in contact with the ground, and the affected ankle joint is located in front of the unaffected ankle joint. In addition, the mid swing phase corresponds to a case where the knee joint is located in front of the ankle.

The terminal swing phase corresponds to a case where the affected foot is separated from the ground, the unaffected foot is in contact with the ground, and the affected ankle joint is located in front of the unaffected ankle joint. In addition, the terminal swing phase corresponds to a case where the knee joint is located behind the ankle.

Hereinafter, regarding the configurations of the knee stretching member 10, the first ankle supporting member 20, and the second ankle supporting member 30, a control method for generating a control force will be described.

The knee stretching member 10 enables the knee joint to be stretched in the terminal swing phase. In addition, the knee stretching member 10 enables a state in which the knee is stretched in the terminal stance phase to be maintained in the loading response phase, thus enabling the affected leg to support body weight.

The first ankle supporting member 20 reinforces the rigidity of the ankle joint in the terminal swing phase in advance against the impact generated when the foot reaches the ground. In addition, in the loading response phase, the first ankle supporting member inhibits excessive inversion and eversion bending of the ankle joint in the terminal stance phase and reinforces the rigidity of the ankle joint in the inversion and eversion directions, thereby absorbing the impact applied to the ankle and improving the gait stability.

The second ankle supporting member 30 assists the motion of the dorsiflexion of the ankle joint in the swing phase so as to inhibit foot drop and enables sufficient foot clearance to be secured.

In addition, the second ankle supporting member 30 assists the motion of the dorsiflexion in the loading response phase so as to absorb the impact applied to the ankle, thus improving the gait stability.

In addition, the second ankle supporting member 30 assists the plantarflexion from the mid swing phase to the pre-swing phase such that thrust is generated while the foot kicks the ground.

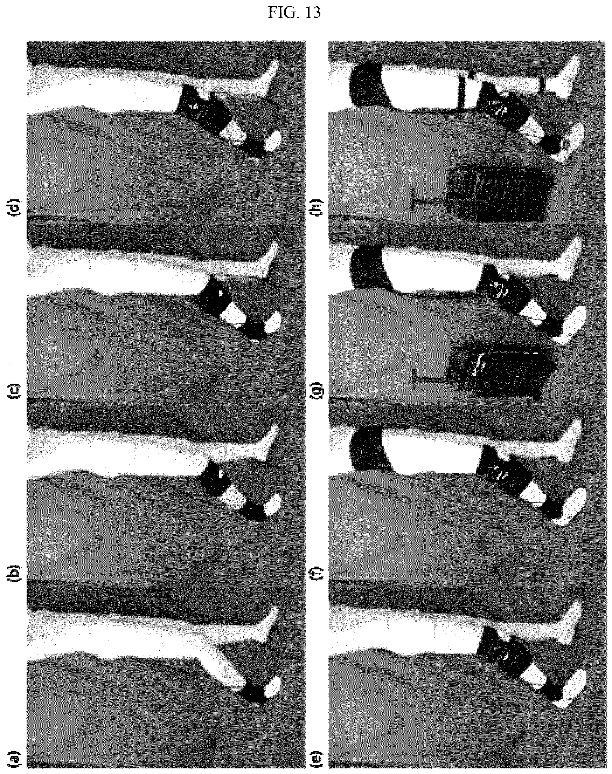

Hereinafter, with reference to a to h in FIG. 13, a wearing order of the robotic leg orthosis 100 for gait rehabilitation training according to the embodiment of the present invention will be described.

Insoles for fixing a plantar pressure sensor and the artificial muscle packs 34 are inserted into indoor shoes. Then, a sensor module is powered up.

With reference to a in FIG. 13, the first ankle supporting member 20 is put on the affected ankle. In addition, the indoor shoes are put on both feet.

Then, with reference to b and c in FIG. 13, the shin sleeve 31 of the second ankle supporting member 30 is put on, the guards 32, to which the artificial muscle packs 34 of the second ankle supporting member 30 are connected, are put on the shin and the calf, and fixing is performed with a Velcro tie.

With reference to d in FIG. 13, the knee stretching member 10 is put on.

With reference to e in FIG. 13, terminal edges of the artificial muscle packs 34 of the second ankle supporting member 30 are connected to a buckle attached to the insole.

With reference to f in FIG. 13, a waist belt, to which the air hose is connected and is branched, is put on.

With reference to g in FIG. 13, the luggage carrier 40 that can perform the autonomous driving and the waist belt are connected to each other with a bundle of air hoses, and air hoses are branched from the bundle of air horses attached to the waist belt so as to be connected to the driving units, respectively.

With reference to h in FIG. 13, the wireless inertial measurement module 50 is attached to at least one of both thighs, shins, and dorsum parts of the feet, and wireless modules for plantar pressure measurement are attached to indoor shoes.

The robotic leg orthosis 100 for gait rehabilitation training described above is not limited to the configurations and methods of the embodiments described above but can have a configuration in which all or a part of individual embodiments are selectively combined such that the embodiment can be variously modified.

It is obvious for those skilled in the art to realize still another specific example within a range not departing from the idea and the essential feature of the present invention. Consequently, the detailed description is not to be construed to be limited in any aspect but is considered an exemplary example.

### Industrial Applicability

According to the embodiments of the present invention, a robotic leg orthosis for gait rehabilitation training, a method for controlling same, and an autonomous driving luggage carrier can assist a paraplegic patient such as a post-stroke hemiplegic patient in a natural gait close to a normal gait according to the patient's gait cycle when the patient walks around. Hence, the robotic leg orthosis can assist a patient in daily life and can maximize rehabilitation effects.

## Claims

1. A robotic leg orthosis for gait rehabilitation training (100), comprising:
an orthosis that is installed on at least one of a knee, an ankle joint, a shin, and a calf of an affected leg which is a rehabilitation target,
wherein the orthosis comprises:
a sleeve surrounding at least one of the knee, the ankle joint, the shin, and the calf; and
an air chamber which is mounted so as to be connected to the sleeve and provides assisting strength to at least one of the knee, the ankle joint, the shin, and the calf, and
**characterised in that**
the robotic leg orthosis for gait rehabilitation training (100) further comprises a luggage carrier (40) that generates compressed air and is capable of supplying the generated compressed air to the air chambers,
wherein the luggage carrier (40) is capable of performing autonomous driving.

2. The robotic leg orthosis for gait rehabilitation training (100) according to claim 1,
wherein the orthosis comprises a first ankle supporting member (20) that is installed at an ankle so as to inhibit inversion or eversion bending of the ankle, and
wherein the first ankle supporting member (20) comprises:
an ankle sleeve (21) surrounding an ankle joint; and
ankle supporting chambers (25) which are air chambers mounted to be connected to the ankle sleeve (21), and which, when air is introduced therein to inflate the air chambers, support the ankle joint so as to inhibit inversion or eversion bending of the ankle.

3. The robotic leg orthosis for gait rehabilitation training (100) according to claim 2,
wherein the air chambers surround both inversion and eversion sides of an ankle, respectively,
wherein air is injected into the air chambers before the affected leg reaches ground, and the air chambers are inflated to provide assisting strength so as to inhibit inversion or eversion bending of the ankle, and
wherein the air chambers maintain pressure therein in a stance phase to provide assisting strength so as to inhibit inversion or eversion bending of the ankle and maintain a stretched state.

4. The robotic leg orthosis for gait rehabilitation training (100) according to any of claims 1 to 3,
wherein the orthosis comprises a second ankle supporting member (30) that is installed on at least one of a shin and a calf such that an ankle is flexed toward a dorsum or a sole of a foot, and
wherein the second ankle supporting member (30) comprises:
a shin sleeve (31) surrounding the shin;
guards (32) that are installed at both the shin and the calf, respectively; and
artificial muscle packs (34) that are connected to the respective guards (32) and have one or more air chambers inside.

5. The robotic leg orthosis for gait rehabilitation training (100) according to claim 4,
wherein the second ankle supporting member (30) further comprises:
a Velcro tie that surrounds and secures the guards; and
a buckle that is installed at a foot accommodating portion and combines the artificial muscle packs (34).

6. The robotic leg orthosis for gait rehabilitation training (100) according to claim 4 or 5,
wherein the air chambers are installed at both sides of the shin and the calf, respectively,
wherein, in a state in which a sole is in contact with ground, air in the air chamber installed at the side of the shin is discharged outside, and air is injected into the air chamber installed at the side of the calf, and
wherein, in a state in which a sole is separated from the ground, air is injected into the air chamber installed at the side of the shin, and air in the air chamber installed at the side of the calf is discharged outside.

7. The robotic leg orthosis for gait rehabilitation training (100) according to any of claims 1 to 6,
wherein the orthosis further comprises a knee stretching member (10) which is provided to be installable on a knee so as to provide assisting strength for enabling a knee to be stretched, and
wherein the knee stretching member (10) comprises:
a knee sleeve (13) surrounding a knee joint; and
knee supporting chambers (15) which are air chambers mounted to be connected to the knee sleeve (13), and which, when air is introduced therein to inflate the air chambers, enable the knee to be stretched by supporting the knee joint.

8. The robotic leg orthosis for gait rehabilitation training (100) according to claim 7,
wherein the air chambers are located at both sides of a rear surface portion of the knee joint, respectively.

9. The robotic leg orthosis for gait rehabilitation training (100) according to claim 7 or 8,
wherein the knee stretching member (10) enables the knee joint to be stretched in a swing phase and enables a state in which the knee is stretched to be maintained in a stance phase.

10. The robotic leg orthosis for gait rehabilitation training (100) according to any of claims 1 to 9,
wherein the sleeve is made of an elastic material, and
wherein the air chambers are made of a soft material.

11. The robotic leg orthosis for gait rehabilitation training (100) according to any of claims 1 to 10,
wherein the luggage carrier (40) comprises: an air compressor (42) which generates compressed air; an air tank (43) which stores the generated compressed air; and a pressure regulator (44) which regulates pressure of the compressed air, and
wherein the luggage carrier (40) comprises a laser distance sensor (41b) which is capable of sensing a distance between a patient and a camera (41a) that is capable of imaging a location of the patient such that the luggage carrier (40) is capable of performing autonomous driving so as to follow the patient by recognizing the location of the patient.

12. A method for controlling the robotic leg orthosis for gait rehabilitation training (100) according to any one of claims 1 to 11, the method comprising:
a step of checking at least one of a rehabilitation patient's gait condition and gait pattern in real time based on an angle between ground and at least one of the patient's thigh, shin, and dorsum of a foot during the rehabilitation patient's walking, by an inertial measurement module (50);
a step of determining the patient's gait cycle as any one of a loading response phase, a mid stance phase, a terminal stance phase, a pre-swing phase, an initial swing phase, a mid swing phase, and a terminal swing phase, based on at least one of the gait condition and the gait pattern, by a main controller (48);
a step of causing a knee stretching member to enable a knee joint to be stretched in the terminal swing phase and enable a state in which the knee is stretched to be maintained for the loading response phase to the terminal stance phase according to the determined gait cycle, by the main controller (48);
a step of causing a first ankle supporting member to start supporting an ankle so as to inhibit inversion or eversion bending of an ankle joint in the terminal swing phase and maintain ankle support so as to inhibit the inversion or eversion bending of the ankle joint for the loading response phase to the terminal stance phase according to the determined gait cycle, by the main controller (48); and
a step of causing a second ankle supporting member to assist dorsiflexion for the initial swing phase to the terminal swing phase, assist dorsiflexion in the loading response phase, and assist plantarflexion for the mid stance phase to the pre-swing phase according to the determined gait cycle, by the main controller (48).

13. A robotic orthosis for rehabilitation training, comprising:
an air chamber that assists a rehabilitation body; and
**characterised in that**
a luggage carrier (40) that generates compressed air and supplies the generated compressed air to the air chamber,
wherein the luggage carrier (40) is an autonomous driving luggage carrier.

14. The robotic orthosis for rehabilitation training according to claim 13, further comprising:
an inertial measurement module (50) that is installed on at least one of a patient's thigh, shin, and dorsum of a foot,
wherein the luggage carrier (40) further comprises a main controller (48), and
wherein the steps of the method for controlling the robotic leg orthosis for gait rehabilitation training (100) according to claim 12 are performed by the inertial measurement module (50) and the main controller (48).

## Patentansprüche

1. Robotische Beinorthese für Gangrehabilitationstraining (100), umfassend:
eine Orthese, die an mindestens einem von einem Knie, einem Knöchelgelenk, einem Schienbein und einer Wade eines betroffenen Beins angebracht wird, das ein Rehabilitationsziel ist,
wobei die Orthese Folgendes umfasst:
eine Manschette, die mindestens eines von dem Knie, dem Knöchelgelenk, dem Schienbein und der Wade umgibt; und
eine Luftkammer, die so montiert ist, dass sie mit der Manschette verbunden ist und mindestens einem von dem Knie, dem Knöchelgelenk, dem Schienbein und der Wade unterstützende Kraft verleiht, und
**dadurch gekennzeichnet, dass** die robotische Beinorthese für Gangrehabilitationstraining (100) ferner einen Gepäckträger (40) umfasst, der Druckluft erzeugt und in der Lage ist, den Luftkammern die erzeugte Druckluft zuzuführen,
wobei der Gepäckträger (40) autonom bewegungsfähig ist.

2. Robotische Beinorthese für Gangrehabilitationstraining (100) nach Anspruch 1,
wobei die Orthese ein erstes Knöchelstützelement (20) umfasst, das an einem Knöchel angebracht wird, um Inversions- oder Eversionsbeugung des Knöchels zu verhindern, und wobei das Knöchelstützelement (20) Folgendes umfasst:
eine Knöchelmanschette (21), die ein Knöchelgelenk umgibt; und
Knöchelstützkammern (25), wobei es sich um Luftkammern handelt, die so montiert sind, dass sie mit der Knöchelmanschette (21) verbunden sind, und die, wenn Luft darin eingeführt wird, um die Luftkammern aufzublasen, das Knöchelgelenk stützen, um Inversions- oder Eversionsbeugung des Knöchels zu verhindern.

3. Robotische Beinorthese für Gangrehabilitationstraining (100) nach Anspruch 2,
wobei die Luftkammern jeweils sowohl die Inversions- als auch die Eversionsseite eines Knöchels umgeben,
wobei Luft in die Luftkammern eingeblasen wird, bevor das betroffene Bein den Boden erreicht, und die Luftkammern aufgeblasen werden, um unterstützende Kraft bereitzustellen, um Inversions- oder Eversionsbeugung des Knöchels zu verhindern, und
wobei die Luftkammern in einer Standphase den Druck darin aufrechterhalten, um unterstützende Kraft bereitzustellen, um Inversions- oder Eversionsbeugung des Knöchels zu verhindern und einen gestreckten Zustand aufrechtzuerhalten.

4. Robotische Beinorthese für Gangrehabilitationstraining (100) nach einem der Ansprüche 1 bis 3,
wobei die Orthese ein zweites Knöchelstützelement (30) umfasst, das an mindestens einem von einem Schienbein oder einer Wade angebracht ist, so dass ein Knöchel in Richtung eines Fußrückens oder einer Fußsohle gebeugt wird, und
wobei das zweite Knöchelstützelement (30) Folgendes umfasst:
eine Schienbeinmanschette (31), die das Schienbein umgibt;
Schutzvorrichtungen (32), die jeweils sowohl an dem Schienbein als auch an der Wade angebracht sind; und
künstliche Muskelpakete (34), die mit den jeweiligen Schutzvorrichtungen (32) verbunden sind und im Inneren eine oder mehrere Luftkammern aufweisen.

5. Robotische Beinorthese für Gangrehabilitationstraining (100) nach Anspruch 4,
wobei das zweite Knöchelstützelement (30) ferner Folgendes umfasst:
ein Klettband, das die Schutzvorrichtungen umgibt und sichert; und
eine Schnalle, die an einem Fußaufnahmeabschnitt angebracht ist und die künstlichen Muskelpakete (34) verbindet.

6. Robotische Beinorthese für Gangrehabilitationstraining (100) nach Anspruch 4 oder 5,
wobei die Luftkammern jeweils an beiden Seiten des Schienbeins und der Wade angebracht sind,
wobei in einem Zustand, in dem eine Sohle mit dem Boden in Kontakt ist, Luft in der an der Seite des Schienbeins angebrachten Luftkammer nach außen abgelassen wird und Luft in die an der Seite der Wade angebrachte Luftkammer eingeblasen wird, und
wobei in einem Zustand, in dem eine Sohle von dem Boden getrennt ist, Luft in die an der Seite des Schienbeins angebrachte Luftkammer eingeblasen wird und Luft in der an der Seite der Wade angebrachten Luftkammer abgelassen wird.

7. Robotische Beinorthese für Gangrehabilitationstraining (100) nach einem der Ansprüche 1 bis 6,
wobei die Orthese ferner ein Kniestreckelement (10) umfasst, das so vorgesehen ist, dass es an einem Knie angebracht werden kann, um unterstützende Kraft bereitzustellen, um Strecken eines Knies zu ermöglichen, und
wobei das Kniestreckelement (10) Folgendes umfasst:
eine Kniemanschette (13), die ein Kniegelenk umgibt; und
Kniestützkammern (15), wobei es sich um Luftkammern handelt, die so montiert sind, dass sie mit der Kniemanschette (13) verbunden sind, und die, wenn Luft darin eingeführt wird, um die Luftkammern aufzublasen, Strecken des Knies ermöglichen, indem das Kniegelenk gestützt wird.

8. Robotische Beinorthese für Gangrehabilitationstraining (100) nach Anspruch 7,
wobei sich die Luftkammern jeweils an beiden Seiten eines hinteren Oberflächenabschnitts des Kniegelenks befinden.

9. Robotische Beinorthese für Gangrehabilitationstraining (100) nach Anspruch 7 oder 8,
wobei das Kniestreckelement (10) in einer Schwungphase Strecken des Kniegelenks ermöglicht und in einer Standphase ermöglicht, dass ein Zustand aufrechterhalten wird, in dem das Knie gestreckt ist.

10. Robotische Beinorthese für Gangrehabilitationstraining (100) nach einem der Ansprüche 1 bis 9,
wobei die Manschette aus einem elastischen Material hergestellt ist und
wobei die Luftkammern aus einem weichen Material hergestellt sind.

11. Robotische Beinorthese für Gangrehabilitationstraining (100) nach einem der Ansprüche 1 bis 10,
wobei der Gepäckträger (40) Folgendes umfasst: einen Luftkompressor (42), der Druckluft erzeugt; einen Luftbehälter (43), der die erzeugte Druckluft speichert; und einen Druckregler (44), der die Druck der Druckluft regelt, und
wobei der Gepäckträger (40) einen Laser-Abstandssensor (41b) umfasst, der zum Messen eines Abstands zwischen einem Patienten und einer Kamera (41a) in der Lage ist, die zum Abbilden einer Position des Patienten in der Lage ist, so dass der Gepäckträger (40) in der Lage ist, sich autonom zu bewegen, um dem Patienten zu folgen, indem er die Position des Patienten erkennt.

12. Verfahren zur Steuerung der robotischen Beinorthese für Gangrehabilitationstraining (100) nach einem der Ansprüche 1 bis 11, wobei das Verfahren Folgendes umfasst:
einen Schritt zum Überprüfen mindestens eines von einem Gangzustand und einem Gangmuster eines Rehabilitationspatienten in Echtzeit basierend auf einem Winkel zwischen dem Boden und mindestens einem von dem Oberschenkel, dem Schienbein und dem Fußrücken des Patienten während des Gehens des Rehabilitationspatienten durch ein Trägheitsmessmodul (50);
einen Schritt zum Bestimmen des Gangzyklus des Patienten durch eine Hauptsteuerung (48) als eine von einer Belastungsreaktionsphase, einer mittleren Standphase, einer terminalen Standphase, einer Vorschwungphase, einer initialen Schwungphase, einer mittleren Schwungphase und einer terminalen Schwungphase basierend auf mindestens einem von dem Gangzustand und dem Gangmuster;
einen Schritt zum Veranlassen eines Kniestreckelements durch die Hauptsteuerung (48), Strecken eines Kniegelenks in der terminalen Schwungphase zu ermöglichen und einen Zustand zu ermöglichen, in dem das Knie gemäß dem bestimmten Gangzyklus für die Belastungsreaktionsphase bis zu der terminalen Standphase gestreckt gehalten wird;
einen Schritt zum Veranlassen eines ersten Knöchelstützelements durch die Hauptsteuerung (48), mit der Unterstützung eines Knöchels zu beginnen, um Inversions- oder Eversionsbeugung eines Knöchelgelenks in der terminalen Schwungphase zu verhindern, und die Knöchelunterstützung aufrechtzuerhalten, um Inversions- oder Eversionsbeugung des Knöchelgelenks für die Belastungsreaktionsphase bis zu der terminalen Standphase gemäß dem ermittelten Gangzyklus zu verhindern; und
einen Schritt zum Veranlassen eines zweiten Knöchelstützelements durch die Hauptsteuerung (48), Dorsalflexion für die initiale Schwungphase bis zu der terminalen Schwungphase zu unterstützen, Dorsalflexion in der Belastungsreaktionsphase zu unterstützen und Plantarflexion für die mittlere Standphase bis zu der Vorschwungphase gemäß dem bestimmten Gangzyklus zu unterstützen.

13. Robotische Beinorthese für Gangrehabilitationstraining, umfassend:
eine Luftkammer, die einen Rehabilitationskörper unterstützt; und
**dadurch gekennzeichnet, dass** ein Gepäckträger (40) Druckluft erzeugt und die erzeugte Druckluft der Luftkammer zuführt,
wobei der Gepäckträger (40) ein autonom bewegungsfähiger Gepäckträger ist.

14. Robotische Beinorthese für Gangrehabilitationstraining nach Anspruch 13, ferner umfassend:
ein Trägheitsmessmodul (50), das auf mindestens einem von einem Oberschenkel, einem Schienbein und einem Fußrücken eines Patienten angebracht ist,
wobei der Gepäckträger (40) ferner eine Hauptsteuerung (48) umfasst und
wobei die Schritte des Verfahrens zum Steuern der robotischen Beinorthese für Gangrehabilitationstraining (100) nach Anspruch 12 von dem Trägheitsmessmodul (50) und der Hauptsteuerung (48) ausgeführt werden.

## Revendications

1. Une orthèse de jambe robotisée pour l'entraînement à la rééducation de la marche (100), comprenant :
une orthèse qui est installée sur au moins un genou, une articulation de la cheville, un tibia et un mollet d'une jambe affectée qui est une cible de rééducation,
dans laquelle l'orthèse comprend :
un manchon entourant au moins un parmi le genou, l'articulation de la cheville, le tibia et le mollet ; et
une chambre à air qui est montée de manière à être reliée au manchon et fournit une force d'assistance à au moins un des genoux, de la cheville, du tibia et du mollet, et
**caractérisée en ce que** l'orthèse de jambe robotique pour l'entraînement à la rééducation de la marche (100) comprend en outre un porte-bagages (40) qui génère de l'air comprimé et est capable de fournir l'air comprimé généré aux chambres à air,
dans lequel le porte-bagages (40) est capable d'effectuer une conduite autonome.

2. L'orthèse de jambe robotique pour l'entraînement à la rééducation de la marche (100) selon la revendication 1,
dans laquelle l'orthèse comprend un premier élément de support de cheville (20) qui est installé au niveau d'une cheville de manière à inhiber la flexion d'inversion ou d'éversion de la cheville, et
dans lequel le premier élément de support de cheville (20) comprend :
un manchon de cheville (21) entourant une articulation de cheville ; et
des chambres de soutien de cheville (25) qui sont des chambres à air montées pour être reliées au manchon de cheville (21), et qui, lorsque de l'air y est introduit pour gonfler les chambres à air, soutiennent l'articulation de la cheville de manière à inhiber la flexion d'inversion ou d'éversion de la cheville.

3. L'orthèse de jambe robotique pour l'entraînement à la rééducation de la marche (100) selon la revendication 2,
dans lequel les chambres à air entourent les côtés d'inversion et d'éversion d'une cheville, respectivement,
dans lequel de l'air est injecté dans les chambres à air avant que la jambe affectée ne touche le sol, et les chambres à air sont gonflées pour fournir une force d'assistance de manière à inhiber la flexion d'inversion ou d'éversion de la cheville, et
dans laquelle les chambres à air maintiennent une pression dans une phase d'appui afin de fournir une force d'assistance de manière à inhiber la flexion d'inversion ou d'éversion de la cheville et à maintenir un état étiré.

4. L'orthèse de jambe robotique pour l'entraînement à la rééducation de la marche (100) selon l'une quelconque des revendications 1 à 3,
dans laquelle l'orthèse comprend un deuxième élément de support de cheville (30) qui est installé sur au moins un parmi un tibia et un mollet de sorte qu'une cheville est fléchie vers un dos ou une plante d'un pied, et
dans laquelle le deuxième élément de support de cheville (30) comprend :
un manchon de tibia (31) entourant les protège-tibias (32) qui sont installés à la fois sur le tibia et le mollet, respectivement ; et des blocs de muscles artificiels (34) qui sont connectés aux protège-tibias respectifs (32) et comportent une ou plusieurs chambres à air à l'intérieur.

5. L'orthèse de jambe robotique pour l'entraînement à la rééducation de la marche (100) selon la revendication 4,
dans lequel le deuxième élément de support de cheville (30) comprend en outre :
une attache Velcro qui entoure et sécurise les protège-tibias ; et
une boucle qui est installée sur une partie de logement de pied et combine les blocs de muscles artificiels (34).

6. L'orthèse de jambe robotique pour l'entraînement à la rééducation de la marche (100) selon la revendication 4 ou 5,
dans laquelle les chambres à air sont installées des deux côtés du tibia et du mollet, respectivement,
dans lequel, dans un état dans lequel une semelle est en contact avec le sol, l'air dans la chambre à air installée sur le côté du tibia est évacué à l'extérieur, et de l'air est injecté dans la chambre à air installée sur le côté du mollet, et
dans lequel, dans un état dans lequel une semelle est séparée du sol, de l'air est injecté dans la chambre à air installée sur le côté du tibia, et de l'air dans la chambre à air installée sur le côté du mollet est évacué à l'extérieur.

7. L'orthèse de jambe robotique pour l'entraînement à la rééducation de la marche (100) selon l'une quelconque des revendications 1 à 6,
dans laquelle l'orthèse comprend en outre un élément d'étirement du genou (10) qui est prévu pour être installé sur un genou de manière à fournir une force d'assistance pour permettre l'étirement du genou, et
dans laquelle l'élément d'étirement du genou (10) comprend :
un manchon de genou (13) entourant une articulation de genou ; et
des chambres de support de genou (15) qui sont des chambres à air montées pour être reliées au manchon de genou (13), et qui, lorsque de l'air y est introduit pour gonfler les chambres à air, permettent au genou d'être étiré en soutenant l'articulation du genou.

8. L'orthèse de jambe robotique pour l'entraînement à la rééducation de la marche (100) selon la revendication 7
dans laquelle les chambres à air sont situées des deux côtés d'une partie de surface arrière de l'articulation du genou, respectivement.

9. L'orthèse de jambe robotique pour l'entraînement à la rééducation de la marche (100) selon la revendication 7 ou 8, dans laquelle l'élément d'étirement du genou (10) permet à l'articulation du genou d'être étirée dans une phase de balancement et permet un état dans lequel le genou est étiré pour être maintenu dans une phase d'appui.

10. L'orthèse de jambe robotique pour l'entraînement à la rééducation de la marche (100) selon l'une quelconque des revendications 1 à 9,
dans laquelle le manchon est fait d'un matériau élastique, et
dans laquelle les chambres à air sont constituées d'un matériau souple.

11. L'orthèse de jambe robotique pour l'entraînement à la rééducation de la marche (100) selon l'une quelconque des revendications 1 à 10,
dans lequel le porte-bagages (40) comprend : un compresseur d'air (42) qui génère de l'air comprimé ; un réservoir d'air (43) qui stocke l'air comprimé généré ; et un régulateur de pression (44) qui régule la pression de l'air comprimé, et
dans lequel le porte-bagages (40) comprend un capteur de distance laser (41b) qui est capable de détecter une distance entre un patient et une caméra (41a) qui est capable d'imager un emplacement du patient de sorte que le porte-bagages (40) est capable d'effectuer une conduite autonome de manière à suivre le patient en reconnaissant l'emplacement du patient.

12. Procédé de commande de l'orthèse de jambe robotique pour l'entraînement à la rééducation de la marche (100) selon l'une quelconque des revendications 1 à 11, le procédé comprenant :
une étape de vérification en temps réel d'au moins une condition de marche et d'un modèle de marche d'un patient en rééducation sur la base d'un angle entre le sol et au moins une cuisse, un tibia et un dos du pied du patient pendant la marche du patient en rééducation, par un module de mesure inertiel (50) ;
une étape de détermination du cycle de marche du patient comme étant l'une quelconque d'une phase de réponse de chargement, d'une phase d'appui médiane, d'une phase d'appui terminale, d'une phase de pré-balancement, d'une phase de balancement initiale, d'une phase de balancement médiane et d'une phase de balancement terminale, sur la base d'au moins une parmi la condition de marche et le modèle de marche, par un contrôleur principal (48) ;
une étape consistant à amener un élément d'étirement du genou à permettre à une articulation du genou d'être étirée dans la phase de balancement terminale et à permettre à un état dans lequel le genou est étiré d'être maintenu pour la phase de réponse de chargement à la phase d'appui terminale selon le cycle de marche déterminé, par le contrôleur principal (48) ;
une étape consistant à amener un premier élément de support de cheville à commencer à soutenir une cheville de manière à inhiber la flexion d'inversion ou d'éversion d'une articulation de cheville dans la phase de balancement terminale et à maintenir le support de cheville de manière à inhiber la flexion d'inversion ou d'éversion de l'articulation de cheville pour la phase de réponse de chargement à la phase d'appui terminale selon le cycle de marche déterminé, par le contrôleur principal (48) ; et
une étape consistant à amener un deuxième élément de support de cheville à assister la dorsiflexion pour la phase de balancement initiale à la phase de balancement terminale, à assister la dorsiflexion dans la phase de réponse à la charge, et à assister la flexion plantaire pour la phase de position intermédiaire à la phase de pré-balancement selon le cycle de marche déterminé, par le contrôleur principal (48).

13. Une orthèse robotique pour l'entraînement à la rééducation, comprenant :
une chambre à air qui assiste un corps de rééducation ; et
**caractérisée par** un porte-bagages (40) qui génère de l'air comprimé et fournit l'air comprimé généré à la chambre à air,
dans lequel le porte-bagages (40) est un porte-bagages à conduite autonome.

14. L'orthèse robotique pour l'entraînement à la rééducation selon la revendication 13, comprenant en outre :
un module de mesure inertiel (50) qui est installé sur au moins l'une des cuisses, tibias et dos d'un pied d'un patient,
dans lequel le porte-bagages (40) comprend en outre un contrôleur principal (48), et
dans lequel les étapes du procédé de commande de l'orthèse de jambe robotique pour l'entraînement à la rééducation de la marche (100) selon la revendication 12 sont réalisées par le module de mesure inertiel (50) et le contrôleur principal (48).
